# EUROPEAN PATENT APPLICATION

(11) **EP 2 783 693 A1**
(43) Date of publication of application: **01.10.2014**
(21) Application number: 12794850.3
(22) Date of filing: 19.10.2012
(51) Int. Cl.: A61K 36/54

(54) **USE OF PEUMUS BOLDUS EXTRACT OBTAINED BY MEANS OF IN VIVO CULTURE AND HAVING ANTI-BOTRYTIS, ALLELOPATHIC AND ANTIOXIDANT ACTIVITY**

(30) Priority: 19.10.2011 CL 26072011
(71) Applicant: Universidad De Santiago De Chile, Santiago (CL)
(72) Inventor: ZUÑIGA, Gustavo, Santiago (CL)
(74) Representative: Isern-Jara, Nuria
(86) International application number: PCT/CL2012/000061
(87) International publication number: WO 2013/056386

(57) **Abstract**

Use of a *Peumus Boldus* extract obtained by *in vitro* culture, with antibotricide, allelochemical and antioxidant capacity.

## Description

### FIELD OF APPLICATION OF THE INVENTION

The present invention is related to the use of a boldo extract obtained by *in vitro* culture. The development includes massively in vitro micropropagating and multiplying *Peumus boldus* and obtaining active extracts. The invention is particularly about a method allowing its production from young shoots of old trees by *in vitro* to produce enough biomass to obtain an enriched extract rich in antibotricide, allelochemical and antioxidant principles useful for controlling *Botrytis cinerea,* weeds and antioxidant for cosmetic use.

### PRIOR ART DESCRIPTION

Plants are a valuable source of secondary metabolites used for the men from ancestral time. Antioxidants are chemical compounds present in many plants and foods and in some cases of synthetic origin Kähkönen, M. P; Hopia, A. I; Vuorela, H. J; Rauha, J; Pihlaja, K; Kujala, T. S; Heinonen, M. (1999) Antioxidant Activity of Plant Extracts Containing Phenolic Compounds J Agric Food Chem 47, 3954 - 3962). Antioxidants have the capability of preventing or delaying the oxidation of an oxidable substrate by different mechanisms, such as free radicals scavening. Synthetic antioxidants have been used as additives in food industry for several decades to preserve the quality of products basically preventing lipid degradation (Pokorny, J and Korezak, J. 2001. Preparation of natural antioxidants. In Antioxidants in foods-practical application. Woodhead Publishing Ltd. Cambridge).

Lipoperoxidation of lipids starts when a polyunsaturated fatty acid (LH) produces free radicals due to the influence of light UV, heat, metals, etc. Free radicals induce the formation of peroxide radicals (LOO.), along with other radical compounds (RC-C.) and peroxide (LOOH) (Shahidi, F. 1997. Natural antioxidants-chemistry, health effects and applications. AOAC Press. Champaign, Illinois, USA).

Antioxidants may prevent lipid peroxidation due to its ability to trap free radicals and lipid peroxidation delay, reducing therefore cellular damage (Halliwel B and Gutteridge J.M.C. 1999. Free radicals in biology and medicine. Third edition. Oxoford University Press.Inc., New York).

The relevance of finding natural antioxidants compounds is because of there is a growing opposition to synthetic antioxidant compounds such as butyl hydroxytoluene (BHT), butyl hydroxyanisole (BHA) y tert-butyl hydroquinone (TBHQ), used as food preservatives. This is because have been reported adverse effects on by these molecules, as for example retard learning in children, possible carcinogenic potential and high cytotoxicity (Proestos, C; Chorianopoulos, N; Nychas, J. E; Komaitis, M. (2005) RP-HPLC Analysis of the Phenolic Compounds of Plant Extracts. Investigation of Their Antioxidant Capacity and Antimicrobial Activity J Agric Food Chem 53, 1190-1195).

To prevent this situation, natural compounds from plants represent a significant supplementary source of antioxidants coming from human diets. They can be used as food preservatives and food supplement to pharmacological levels (Proestos, C; Chorianopoulos, N; Nychas, J. E; Komaitis, M. (2005) RP-HPLC Analysis of the Phenolic Compounds of Plant Extracts. Investigation of Their Antioxidant Capacity and Antimicrobial Activity J Agric Food Chem 53, 1190-1195). 8, 9).

Polyphenols correspond to a large group of wide compound diversity, whose main feature showing an aromatic ring in their structures, which has one or more hydroxyl substituents (Vermeris W and Nicholson R. 2008. Phenolic compound biochemistry. Springer Science Business Media B.V., Berlin). They are synthesized during the secondary metabolism of plants. There are a variety of these compounds distributed along to the whole plant kingdom and also in fungi. The role of these compounds within the plant organism is not entirely clear, but in some cases they act as pigments such as the case of anthocyanins and anthocyanidins, which benefit the attraction of pollinating agents to the plants, and also a function of protection against ultraviolet rays (Bravo L. 1998. Polyphenols: chemistry, dietary sources, metabolism, and nutritional significance. Nutritions Reviews 56:317).

However, its action is not only for protection but also they are used as communication environmental mean, phenomena known as allelopathy (Zhao-Hui Li, Qiang Wang, Xiao Ruan, Cun-De Pan and De-An Jiang. 2010. Phenolics and Plant Allelopathy, Molecules 15, 8933-8952). Through secondary metabolites plants are able to modulate its environment and to show a defense against of the aggression of different plants, pathogens and herbivores (Zhao-Hui Li, Qiang Wang, Xiao Ruan, Cun-De Pan and De-An Jiang. 2010. Phenolics and Plant Allelopathy, Molecules 15, 8933-8952).

Biosynthesis of these compounds is produced from "shikimic" acid and acetate pathways. They are stored into vacuoles and mainly accumulated in epidermal cell layers. They are found mainly in glycosylated form, this linkage with sugars increase its polar features facilitating its transport inside the plant and its storage into vacuoles (Vermeris W and Nicholson R. 2008. Phenolic compound biochemistry. Springer Science Business Media B.V., Berlin).

Phenolic compounds have a high ability to act as free radical scavengers, because of the structural features in the molecule allow the formation of a radical which is much more stable than its antecessor by acting as unpaired electron acceptor of a free radical, this because the electron is permanently relocated due to the presence of multiple molecular double bonds (Vermeris W and Nicholson R. 2008. Phenolic compound biochemistry. Springer Science Business Media B.V., Berlin).

Medicinal plants represent a significant source of antioxidants principles particularly the Chilean endemic species. Within these species, it is remarked the position of boldo, *Peumus boldus,* whose use as medicinal plant has been registered for several pharmacopeias and its use inside the country has been described from several centuries (Quezada N, Asencio M, Del Valle J, Aguilera JM, Gomez B. 2004. Antioxidant activity of crude extract, alcaloid fraction, and flavonoid fraction from Boldo (Peumus boldus Molina) leaves. J Food Sci 69: 371-376.). The main use of this plant is as leaf infusion and is administered to cure liver problems since it is choleretic and cholagogue.

The medicinal plants represent an important source of antioxidants principles, in particular the species endemic to Chile. Among these species, highlights the boldo, Peumus boldus, whose use as a medicinal plant is registered in various pharmacopoeias and use in the country for centuries described (Quezada N, Asencio M, Del Valle J, Aguilera JM, Gomez B. 2004. Antioxidant activity of crude extract, alcaloid fraction, and flavonoid fraction from Boldo (Peumus boldus Molina) leaves. J Food Sci 69: 371 376.). The main use of this plant as an infusion of its leaves and is used to treat liver complaints, as it is choleretic and cholagogue

It has been also described that boldo has anti-inflammatory, antioxidants and sedative features (Speisky H, Cassels B. Boldo and boldine: an emerging case of natural drug development. Pharmacological Research 1994; 29:1-12)

Among the compounds described in boldo leaves are found essential oils, isoprenoids, flavonoids and about 17 alkaloids, being boldina the majority. Boldina is accumulated in a greater ratio in the bark (Speisky H, Cassels B. Boldo and boldine: an emerging case of natural drug development. Pharmacological Research 1994; 29:1-12). It has been described that boldine shows antioxidant capabilities which would partially explain the medicinal properties of boldo (Speisky H, Cassels B, Lissi E, Videla L. Antioxidant properties of the alkaloid boldine in systems undergoing lipid peroxidation and enzyme inactivation. Biochemical Pharmacology 1991; 41:1575-1581.).

Boldo is traditionally used as firewood and charcoal, frequently looks a shrub habit due to its capability of producing new shoots after cutting or fire (Del Fierro, P., L. Pancel. Experiencia silvicultural del bosque nativo de Chile. Santiago: Publicaciones Lo Castillo, 1998 3). The main interest is focused in its leaves which are harvested in summer for marketin and export. Currently boldo exploitation is based on wild recollection, then domestication and culture studies have been started to this specie with the finality of reducing the pressure on natural ecosystems and achieve an improvement in the quality of the harvested product (Vogel, H., I. Razmilic, U. Doll. Contenido de aceite esencial y alcaloides en diferentes poblaciones de boldo (Peumus boldus Mol.). Ciencia e investigación agraria (Chile), 1997, vol. 24, Nº 1, p. 1-6.).

Recently, the *in vitro* boldo culture has been described by means of direct organogenesis (Ríos D, Sandoval D and Gómez C 2010, In vitro culture of Peumus boldus Molina via direct organogénesis. Molecular Medicinal Chemistry. Vol. 21 January-April 2010, 70-72). However, hormone combinations used are completely different. Further, in this work, the antioxidant capability has not been evaluated neither the metabolite profile was characterized in seedlings. Thus, in the prior art is unknown the metabolite profile produced by seedlings *in vitro.*

For this reason, it is necessary to find an alternative to allow the generation of boldo biomass without having negative effects over the specie. The *in vitro* tissue culture represents a highly alternative for the constant production of plant material, wichh leads the continuous production of plant biomass generating natural products without a risk to the germoplasm conservation (Moran G., Colque R, Viladomat F., Bastida, J. y Codina C. 2003. Masspropagation of Cyrtanthus clavatus and Cyrtanthus spiralis using liquid medium culture. Scientia Horticulturae 98: 49 - 60 17). Furthermore, this technique also eliminates unfavorable factors such as crop contamination, since it is carried out under sterile conditions. Also it is eliminated the effect of season due to it is cultured under standardized environmental conditions which would allow a continuous production. Several studies have demonstrated that plant tissue coming from *in vitro* tissue is able to produce specific compounds in similar concentrations than intact plants under in vivo conditions. Thus the remarkable advances in the last decade in the plant biotechnological area have given the opportunity that plants are considered as authentic bioreactors to the production of the interested secondary metabolites.

The obtention of extracts with biological properties from boldo plants grown under wild conditions presents as main limitation the big variability induced by the environment. Similarly, it has not been described that boldo plants cultured *in vitro,* produce extracts with biological activity.

The object of the invention is to obtain a boldo extract from *in vitro* culture with antioxidant,allelopathic and fungicide properties, in particular standardized based on their content in boldine.

The objective of the invention is obtaining a boldo extract from an *in vitro* culture with antioxidants, allelopathic and fungal properties, particularly one standardized in function of its boldine content.

Another objective of the invention is the plant material obtained by *in vitro* culture of *P.*

### boldus

Another object of the invention is a standardized antioxidant boldo extract.

Another object of the invention is the obtention of a standardized allelopathic boldo extract.

Another object of the invention is the obtention of a standardized antifungal boldo extract.

### DESCRIPTION OF FIGURES

**FIGURE 1** depicts the features of microseedlings of *Peumus boldus* cultured *in vitro.* Boldo seedlings cultured *in vitro,* are used as raw material to obtain extracts. Seedlings are used between 30 to 45 days of culture.
**FIGURE 2** shows the effect of solvent in the extraction of active principles. The presence of active principles is determined evaluating the antioxidant capacity of the extract. The extract is obtained by simple diffusion in 85% ethanol or hot water. Extracts are prepared in the following relationship always 1 g fresh tissue in 10 volumes of solvent.
**FIGURE 3** it is shown the effect of the type of extraction in the antioxidant activity of the boldo extract. Diffusion: Seedlings are contacted with solvent (ethanol 85%), for at least 72 hrs. Maceration. Seedlings are ground under liquid nitrogen and then one volume of ethanol 85% is added. The mixture is kept in rest during 15 minutes and then it is filtered. The work is carried out with the supernadant. Sonication. Seedlings are located in ethanol 85% and then sonicated during 2 hours (50/60 Hz).
**FIGURE 4** shows the antioxidant capability of *in vitro* boldo extracts as function of the culture age. Antioxidant capacity is showed for cultured plants under different times. The extract is obtained by sonication in ethanol at a ratio of 1 g of tissue by 10 solvent volumes.
**FIGURE 5** shows the variation of the phenolic compound content in *in vitro* boldo extracts as function of the culture age. Phenol content is expressed in galic acid (GA) equivalents.
**FIGURE 6** shows the effect of the *in vitro* boldo extract in the germination of seeds. The effect of an extract prepared in ethanol at a ratio of 1 g of tissue in 10 solvent volumes was evaluated. The germination percentage of seed submitted to different concentrations of boldo extract was performed under controlled conditions at 25°C.
**FIGURE 7** shows the effect of the *in vitro* boldo extract in the development of fungus *Botrytis cinerea.* A. growth of *B*. *cinerea* in plates with control culture media and culture media supplemented with boldo extract at 4%. B. growth of *B*. *cinerea* in control liquid media (blue line) and two *in vitro* boldo extract concentrations (yellow 2% and pink 4%),
**FIGURE 8** shows the variation of the principal compounds present in the *in vitro* boldo extracts. The content of some principal compounds is determined using High Performance Liquid Chromatography with a diode detector (HPLC-DAD).
**FIGURE 9** shows the chromatogram of analysis of *in vitro* boldo extract by means of liquid chromatography with mass detector (LC/MS).

### INVENTION DESCRIPTION.

*Peumus boldus* is one of the Chilean medicinal plants which are more extensively acknowledged abroad. Popular medicine mainly uses the leaves of this plant to digestive or liver conditions and less frequently to kidney diseases; it is acknowledged by dissolving liver and kidney stones; also it is used to stimulate or sedate. As poultice and bath is used to mitigate neuralgic and rheumatic pains.

Such properties are due to the chemical components forming part of the plant, which includes essential oils enriched in monoterpenic hydrocarbons (p-cymene, a-pinene), oxygenated monoterpenes (ascaridole, cineol or eucalyptol, linalol, among others), and isoquinoleinic alkaloids type aporphine (mainly boldine). To this is added the percentage of flavonids, catechin, cumarines and resins that also form part of the Boldo active principles. The effect as *in vitro* antioxidants is known to the boldo components. The antioxidant potential of an aqueous boldo extract is based on significant compounds such as flavonoids and catechins which are preserved because of both of these compounds are water soluble (Del Valle J M, Rogalinski T, Zetzl C, Brunner G. Extraction of boldo (Peumus boldus M.) leaves supercritical CO and hot pressurized water. Food Research International 2005; 38 (2): 203- 213). Several studies demonstrate the cholagogues and choleretics effects of boldo in animals and further it is observed that the toxic dose to a mouse is 1 g/kg by weight (The Europian Agency for the Evaluation of Medicinal Products Veterinary Medicines Evaluation UnitEMEA/MRL/548/99 - final Julio 1999).

According to Germany pharmacopeia, boldo is broadly used in compositions to liver diseases as well as it has also been described its use in the treatment of cellulites. All this associated to its antioxidant capability. The *in vitro* plant tissue culture, is a useful response to the current needs of continuous production of active metabolites. This application claims a process to obtain a plant composition having antioxidant capacity. This process is surprising since an excellent support formulation has been found to the *in vitro* culture to obtain a maximum of active metabolites (total phenols) in 21 to 30 days. These extracts are standardized by the main components that are syringic acid (EC50 0.17 mg/ml) and chlorogenic acid (EC50= 0.49), then this process demonstrates that it is an excellent alternative to industrial use and its incorporation in phytopharmaceutic, cosmetic and food compositions.

The present invention presents a system of *in vitro* multiplication of boldo biomass, producing bioactive principles. It has been developed a system of nutrients allowing the biomass preservation, growing and multiplication. Further, it is contemplated the use of elicitors to regulate the production of active principles.

### DETAILED DESCRIPTION OF THE INVENTION.

### EXAMPLE N° 1. Process to obtain microseedlings of Peumus boldus in an in vitro culture

In a first phase mother material is collected. Mother plants, which are used in this study, come from Cuesta Barriga, Region Metropolitana. New shoots collected from adult trees were transported to the laboratory and prepared for an introduction into an *in vitro* culture system. Shoots were washed with copious water and then disinfected with an antifungal solution. After the washing into a laminar flow chamber the explant were located into vessels of 250 mL of culture media. MS with different hormone combinations (Benzylaminepurine (BAP) and/or kinetine (K) and indolbutiric acid (IBA). Starting media and explants multiplication of boldo are indicated Table 1.

**Table 1. Compositions of culture media used to micropropagate Peumus boldus**

| 1 a) | |
|---|---|
| **MS Culture Media Ingredients** | **Concentration** |
| Basal MS MEdia (PhytoTech Lab TM) | 4.43 gr/L |
| Sucrose | 20 gr/L |
| Benzyl amine purine (BAP) | 0.1-1.0 mg/L |
| Indolbutiric acid (IBA) | 0.1 -0.5 mg/L |
| Biotine | 0.01 g/mL |
| Polyvynylpyrrolidone (PVP) | 100 mg/L |
| NaOH/NaCl enough amount (qs) to adjust pH | pH 5.6 - 5.7 |
| Bidistillated water qs | 1000 ml |

| 1 b) | |
|---|---|
| **MS Culture Media Ingredients** | **Concentration** |
| Basal MS Media (PhytoTech Lab TM) | 4.43 gr/L |
| Sucrose | 30 gr/L |
| Adenine Isopentenyl (2IP) | 0.1-1.0 mg/L |
| PVP | 100 mg/L |
| NaOH/NaCl qs to adjust pH | pH 5.6 - 5.7 |
| Bidistillated qs | 1000 ml |

| 1c) | |
|---|---|
| **MS Culture Media Ingredients** | **Concentration** |
| Basal MS Media (PhytoTech Lab TM) | 4.43 gr/L |
| Sucrose | 20 gr/L |
| Benzyl amine purine (BAP) | 0.2-1.0 mg/L |
| Indolbutyric Acid (IBA) | 0.1 -0.5 mg/L |
| PVP | 100 mg/L |
| NaOH/NaCl qs to adjust pH | pH 5.6 - 5.7 |
| Bidistillated water qs | 1000 ml |

| 1d) | |
|---|---|
| **MS Culture Media Ingredients** | **Concentration** |
| MS Basal Media (PhytoTech Lab TM) | 4.43 gr/L |
| Sucrose | 30 gr/L |
| Adenine Isopentenyl (2IP) | 0.1-1.0 mg/L |
| PVP | 100 mg/L |
| NaOH/NaCl qs to adjust pH | pH 5.6 - 5.7 |
| Bidistillated water qs | 1000 ml |

The composition of the culture media *in vitro* Murashige y Skoog (MS media MS) used in this process is broadly known in the prior art.

The present process of *in vitro* boldo culture permits to obtain a boldo extract which has not been described in the state of the art. The process consists on the use of the tissue culture technique in that young nodal segments of adult tree are used. The nodal segments generate shoots. This process permits producing a boldo biomass which is enough to generate antioxidants extracts.

### EXAMPLE N° 2. BIOMASS DUPLICATION.

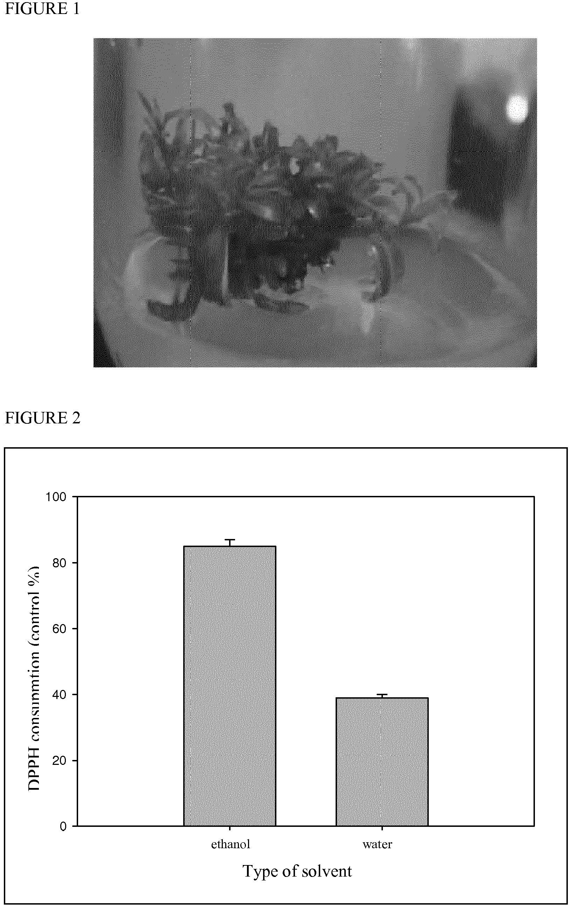

Biomass development into vessels is shown in Figure 1. 3 P. Boldus explants (shoots) as obtained in example 1 were inoculated in each vessel. As culture media to the propagation step, the MS basal medium was used (Table 1, Example 1) which was supplemented with sucrose 2% p/v, kinetine 0.1 mg/L, BAP 0.3 mg/L and biotine 0.1 mg/L. The pH of the culture media was 5.6 - 5.7. After 1 month of culture each explants have been generated 5 new shoots (Figure 1).

### EXAMPLE N° 3. KINETIC OF TOTAL PHENOL INDUCTION AND ANTIOXIDANT BOIOLOGICAL ACTIVITY.

The synthesis of secondary metabolites with biological properties is one of the main points of interest in the present process. An evaluation of total phenolic compounds and the antioxidant biological activity of *Peumus boldus* plants are illustrated. Figure 2 shows the effect of the solvent in the extraction of active principles. The presence of active principles is determined evaluating the antioxidant capacity of the extract. The extract is obtained by simple diffusion in ethanol 85% or heated water. Extract are always prepared in the following relation 1 g of fresh tissue in 10 volumes of solvent. It can be concluded that ethanol is a better solvent to extract the active principles from the *in vitro* plants.

Effect of the type of extraction in the antioxidant capability of boldo extracts.

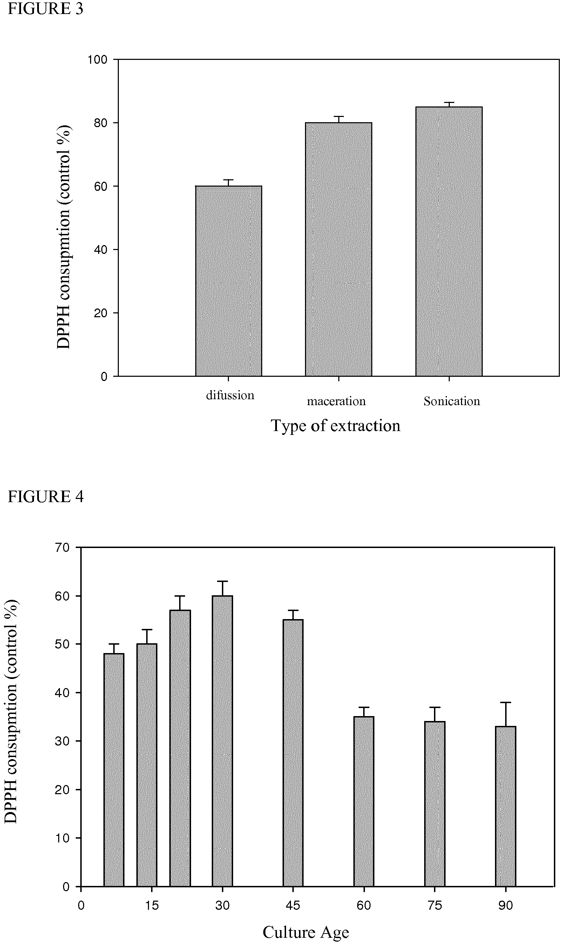

In Figure 3, it is shown the effect of the type of extraction in the antioxidant activity of the extract. Diffusion: seedlings are contacted with the solvent (ethanol 85%), for at least 72 hrs. Maceration. Seedlings are ground in liquid nitrogen and then a volume of ethanol 85% is added. The mixture is allowed to rest during 15 minutes and then it is filtered. The work is carried out with the supernandant. Sonication. Seedlings are placed in ethanol 85% and then sonicated during 2 hours (50/60 Hz).

Figure 4 shows the scavenging capacity on the free radical DPPH during the cultures period evaluated. It is shown the antioxidant capacity of extracts obtained from plants cultured in different times. The extract was obtained by sonication in ethanol 85% at a ratio of 1 g of tissue in 10 volumes of solvent.

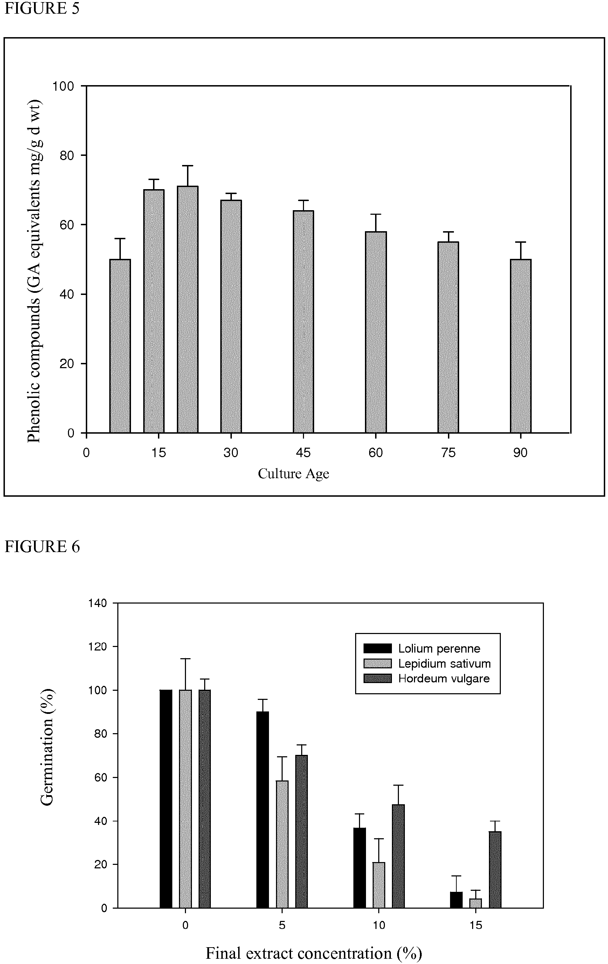

Figure 5 shows the variation in the content of phenolic compounds with antioxidant activity in boldo extracts Phenolic compound content was determined by using the Folin-Ciocalteu reagent.

### EXAMPLE N°4. OTHER BIOLOGICAL ACTIVITIES OF BOLDO EXTRACT.

Figure 6 illustrates the allelophatic activity of boldo extract. The effect of different concentracions of boldo extract on the germination of seeds of *Lepidium sativum, Hordeum vulgare* and *Lolium perenne* are determined. In all cases the percentage of germination decreases with increasing concentration of *in vitro* boldo extract.

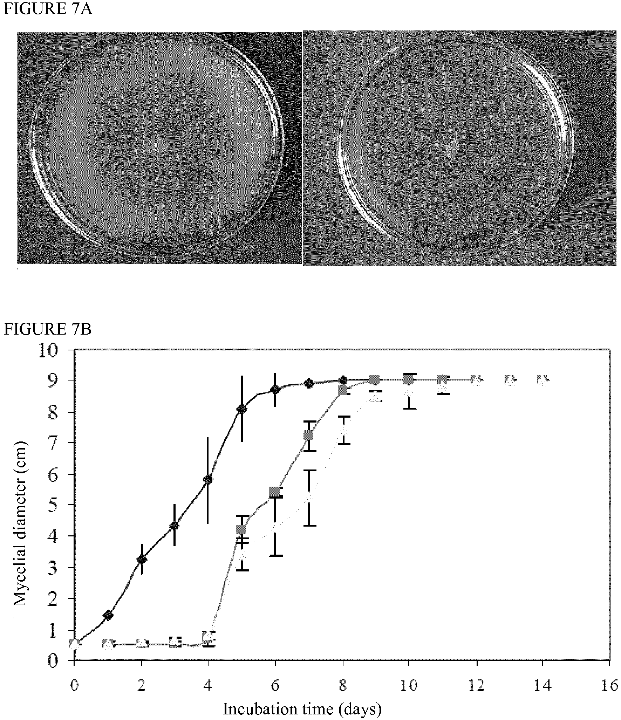

Other biological activities of *in vitro* cultured boldo extract were evaluated. Figure 7A shows the effect of *in vitro* boldo extract on the mycelial growth of Botrytis cinerea in standard media supplemented with 4% of *in vitro* boldo extract. It is noted that the mycelial growth is inhibited in the presence of boldo extract. Figure 7B shows kinetics of fungus growth at 2 concentrations of *in vitro* boldo extract (2 and 4%). The presence of extract in the culture media inhibited the fungal growth.

### EXAMPLE N°5. CHARACTERIZATION OF BOLDO EXTARCT.

Boldo extract obtained from *in vitro* seedlings, was characterized by liquid chromatography with diode array detector (HPLC-DAD) and liquid chromatography with mass detector (LC-MS).

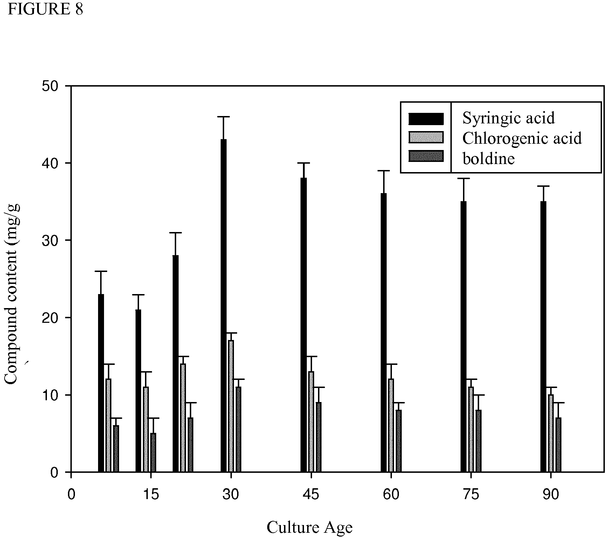

Figure 8 shows the variation in the content of major compounds identified by HPLC-DAD.

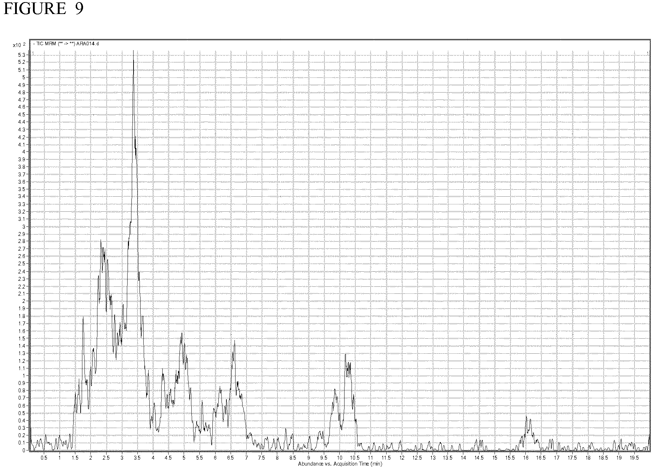

Figure 9 shows the relative concentration of each of the compounds detected by LC-MS.. This technique of high sensibility allowed the detection of other metabolites which were present in the extract, which were not identified in the analysis by HPLC-DAD. However, similarly to the analysis by HPLC-DAD the main compounds are chlorogenic, syringic and p-cumaric acids. This analysis allowed to determine that the boldine is present as glucoside. Table 2 shows the main phenolic compounds and a percent concentration into the *in vitro* boldo extract.

| **Compound** | **%** |
|---|---|
| Rutin | 0.9 |
| Apigenin-glu | 1.4 |
| Rosmarinic acid | 3.7 |
| Chlorogenic acid | 20.0 |
| Caffeic acid hexoside | 8.9 |
| Carnosol | 2.1 |
| Hydrobenzoic acid | 4.3 |
| Gallic acid | 5.4 |
| Vanillic acid | 3.3 |
| Cumaric acid | 8.0 |
| Protocathequic acid | 3.2 |
| Quercetin hexoside | 4.4 |
| Quercitrin | 5.4 |
| luteoline glu | 3.2 |
| Kaempferol 3-Glu | 1.2 |
| Syringic Acid | 24.7 |

## Claims

1. Extract of *Peumus boldus* with antibotricide, antioxidant and allelophatic capacity *wherein*:
a) extract is obtained from shoots cultured *in vitro* which are obtained from adult trees shoots,
b) extract is obtained using seedlings of between 21 and 45 days of culture,
c) extract contains a metabolite mixture of phenolic compounds and alkaloids.

2. The boldo extract according to claim 1, which contains rutin, apigenine 7-O-rutinoside, rosmarinic acid, chlorogenic acid, syringic acid, hexasise caffeic acid, carnosol, hydroxybenzoic acid, gallic acid, boldine and glucoside boldine.

3. The boldo extract according to claim 1, where the main phenolic compounds are chlorogenic acid (20%), syringic acid (24.7%) and cumaric acid (8%).

4. The boldo extract according to claim 1, which contains boldine (12%) and glucoside boldine (23%).

5. The boldo extract according to claim 1, which is obtained extracting with an extraction solvent selected from hydroalcoholic solutions between 70-85%.

6. The boldo extract according to claim 1, which is obtained by diffusion, maceration or sonication.

7. The boldo extract according to claim 2, which is obtained extracting by diffusion between 12 to 72 hrs, at 22 °C.

8. The boldo extract according to claim 2, where the extract is obtained by maceration, grinding in liquid nitrogen, adding 85% ethanol and filtered after 5 to 15 minutes to separate the supernatant

9. The boldo extract according to claim 2, which is obtained extracting by sonication, contacting the shoots with ethanol 85% and sonicated for 1 to 3 hours, between 50 to 60 Hz, at 40 °C.

10. Use of the extract according to any one of claims 1 to 9, useful as antifungal to the *Botrytis cinerea* control.

11. Use of the extract according to any one of claims 1 to 9, useful as allelophatic to herbicidal preparations.

12. Use of the extract according to any one of claims 1 to 9, useful as antioxidant in cosmetic preparations.

## Amended claims

### Amended claims under Art. 19.1 PCT

1. Extract of *Peumus boldus* with antibotricide, antioxidant and allelophatic capacity *wherein*:
a) extract is obtained from shoots cultured *in vitro* which are obtained from adult trees shoots, by:
a.1) maceration, grinding in liquid nitrogen, adding 85% ethanol and filtered after 5 to 15 minutes to separate the supernatant; or
a.2) sonication, contacting the shoots with ethanol 85% and sonicated for 1 to 3 hours, between 50 to 60 Hz, at 40°C
b) extract is obtained using seedlings of between 21 and 45 days of culture,
c) extract contains a metabolite mixture of phenolic compounds and alkaloids.

2. The boldo extract according to claim 1, which contains rutin, apigenine 7-0-rutinoside, rosmarinic acid, chlorogenic acid, syringic acid, hexasise caffeic acid, carnosol, hydroxybenzoic acid, gallic acid, boldine and glucoside boldine.

3. The boldo extract according to claim 1, where the main phenolic compounds are chlorogenic acid (20%), syringic acid (24.7%) and cumaric acid (8%).

4. The boldo extract according to claim 1, which contains boldine (12%) and glucoside boldine (23%).

5. Use of the extract according to any one of claims 1 to 4, useful as antifungal to the *Botrytis cinerea* control.

6. Use of the extract according to any one of claims 1 to 5, useful as allelophatic to herbicidal preparations.

7. Use of the extract according to any one of claims 1 to 5, useful as antioxidant in cosmetic preparations.

Statement under Art. 19.1 PCT
To point out the differences between the invention and the cited prior art, the applicant remarks that D1 describes antioxidants properties of boldine, alkaloid presents in boldus. The form in which boldine is obtained is totally different from the one used in the present case. Then, it is not evident that such extract shows the same properties.

D2 mainly is related to the effects of boldine, main alkaloid presents in boldus, as antioxidant. It is not related to the present extract containing between other components boldine in pure form.

D3 is related to the protecting effect of erythrocytes against free radical attack. D3 is related to a pure molecule but not an extract of different molecules.

D4 is related to the herbicide effect of boldus essential oils. Essential oils are formed by molecules having a polarity different from the ones extracted with polar solvents as ethanol. Thus, it is not evident that an extract as the present one has similar biological activities.

D5 reports the fungal activity of boldus essential oils. The oil composition reported does not have anything in common with the present extract as previously mentioned to D4.

In summary, the documents reviewed are related to boldine or boldus essential oils. The present extract is related to an extract obtained using as solvent ethanol, which extracts preferably molecules of polar nature and glycosides that differs from a composition of essential oils.

The above mentioned can be clear in the following table which has been taken from the document XP009167444.

**Table 1. Main components of the essential oils from Peumus boldus, Laurellopsis philippiana, and Laurella sempervirens.**

| **Compound** | ***Peumus boldus*** | ***Laurellopsis philippiana*** | ***Laurella sempervirens*** |
|---|---|---|---|
| | % | | |
| Ascaridole | 34.80 | - | - |
| 3-Carene | 1.81 | 53.81 | 1.97 |
| α-Phellandrene | 1.01 | 2.95 | 0.92 |
| β-Phellandrene | 5.43 | - | 1.98 |
| 1,2-Dimethoxy-4-(2-propenyl)-phenol | - | 10.58 | - |
| Safrole | - | 2.33 | 69.30 |
| αα-1-Methyl-3-cyclohexane | - | 5.86 | |
| α-Pinene | 3.19 | 2.30 | 0.39 |
| β-Pinene | 1.02 | - | - |
| Limonene | 16.10 | - | - |
| Encalyptol (cineole) | 11.95 | 14.76 | - |
| α-Terpineol | 8.90 | - | - |
| β-Myrcene | 0.37 | - | - |
| p-Cymol | 7.85 | - | - |
| Linalool | 1.03 | - | - |
| Nerolidol | 4.95 | - | - |
| α-1-Methyl-3-cyclohexadiene | - | 5.14 | - |
| 1-Methyl-4-1-methyl ethyl cyclohexane | - | - | 18.55 |
| Other components | 1.59 | 2.27 | 6.89 |

To clarify the above mentioned claims have been amended to introduce the essential condition of extracting with ethanol as solvent.
